# EUROPEAN PATENT APPLICATION

(11) **EP 4 104 889 A1**
(43) Date of publication of application: **21.12.2022**
(21) Application number: 21754312.3
(22) Date of filing: 05.02.2021
(51) Int. Cl.: A61M 29/02, A61M 25/10, A61M 31/00

(54) **MULTIFUNCTIONAL BALLOON DILATATION CATHETER FOR INTRACORPOREAL MEMBRANE OXYGENATION AND USAGE METHOD THEREFOR**

(30) Priority: 16.02.2020 US 202062977325 P
(71) Applicant: Dongguan TT Medical Inc., Dongguan, Guangdong 523808 (CN)
(72) Inventor: WU, Tim, Dongguan, Guangdong 523808 (CN); BI, Yuying, Guangdong, Guangdong 523808 (CN)
(74) Representative: Vitina, Maruta
(86) International application number: PCT/CN2021/075458
(87) International publication number: WO 2021/160030

(57) **Abstract**

Disclosed is a multifunctional balloon dilatation catheter for intracorporeal membrane oxygenation, comprising: a catheter hub, a dilatation balloon, and M channels. The M channels comprise at least a balloon dilatation channelfor dilating the narrowed part of the airway, a positioning & detection channelfor positioning or detecting while inserting, a supply channel for continuous oxygen supply or drug administration to the patient,and a secretion removal channelfor removing secretions from the airway. The catheter hub is a multi-channel connection device, through which theM channels are respectively connected to the external therapeutic devices of the catheter. The catheter is inserted into a bronchiole of the lungs. When using the catheter for emergency treatment of patients with pneumonia, especially COVID-19, measures such as airway dilatation, oxygen supply, sputum suction and biopsy can be simultaneously performed, thereby improving the survival rate of patients and reducing lung damage.

## Description

### CROSS-REFERENCE TO RELATED APPLICATIONS

This application claims priority to the U.S. Patent Application No. 62977325(Intracorporeal Membrane Oxygenation Balloon Catheter System for Rapid Treatment of Novel Coronavirus Disease (COVID-19)), filed on February 16, 2020. The content of the aforementioned application, including any intervening amendments thereto, is incorporated herein by reference in its entirety.

### TECHNICAL FIELD

The present invention relates to the field of membrane oxygenation, and in particular, to a multifunctional balloon dilatation catheter for intracorporeal membrane oxygenation and usage method therefor.

### BACKGROUND OF THE INVENTION

Since December 2019, the novel coronavirus disease (COVID-19) pandemic has successively broken out around the world, becoming a major medical issue that needs to be solved urgently. It is caused by severe acute respiratory syndrome coronavirus 2 (SARS-CoV-2), which binds to the host cell receptor, angiotensin-converting enzyme 2 (ACE2), in mucosal tissues after entering the human body mainly through the respiratory and digestive tracts. The ACE2 receptors are widely found on various mucosal cells of human body. After the virus invades the human body, it incubates for 2-3 weeks, and then rapidly reproduces and stimulates the patient's autoimmune mechanism to develop the disease. The initial symptoms is similar to those of the common cold. If it is not effectively controlled, about 20% of patients become severe in about 1 week.

Severe cases are mainly caused by rapid respiratory failure due to the cytokine storm in the lungs, with the clinical manifestations like extreme dyspnoea and rapid decline in blood oxygen saturation. CT images show localized (early-stage) or diffuse (advanced-stage) patchy opacities (white lung(s)) in both lungs. Pathology shows that the patient's lungs begin to have an inflammatory reaction from the end, and a large number of viscous inflammatory secretions covers the bronchioles and alveoli (forming the white lung(s) seen in the CT images), blocking the normal gas exchange of the membrane, and then resulting in rapid drop in blood oxygen saturation. Hypoxia further aggravates the damage of other organs, especially in the elderly or patients with other diseases at the same time.

Currently, the treatment methods for COVID-19 are not perfect, and supportive treatment is mainly used in clinical practice. Only mechanical oxygenation and extracorporeal membrane oxygenation (ECMO) are used for rescuing the critically ill patients, and the success rate is very low. The latest pathological analysis results show that a large amount of viscous secretions accumulated in the lungs of critically ill patients obstruct the airway, hinder the ventilation function of the lungs, resulting in hypoxia due to mechanically delivered oxygen unable to reach the terminal bronchioles. Long-term hypoxia of alveoli and terminals leads to alveolar damage and ventilation dysfunction.

Currently, the endotracheal intubation and the ventilator are commonly used for maintenance therapy. When a patient is in respiratory distress and his/her oxygen saturation decreases, assisted breathing becomes necessary. Due to the increase of mucin secreted by the epidermal cells of the respiratory tract in the late stage of COVID-19, the viscous secretions obstruct the airway, so that the supplied oxygen cannot effectively reach the alveoli for gas exchange, resulting in alveolar damage. In addition, high-flow hyperbaric oxygen may push the blocked viscous secretions in the patient's airway into the lower respiratory tract to form a mucous plug, which further blocks the micro bronchioles and may lead to bacterial infection. Therefore, it is necessary to dilute, dissolve and remove viscous secretions in the patient's airway before oxygen therapy.

Extracorporeal membrane oxygenation (ECMO) is commonly used for emergency treatment. When the ventilator cannot maintain the patient's blood oxygen level, the patient needs to be transferred to the ICU for ECMO. ECMO is the last resort for a patient who cannot effectively receive mechanical ventilation for a variety of reasons. When it is in use, the blood is pumped from the punctured femoral vein of the patient. The hypoxic blood is artificially oxygenated by the ECMO machine and the neck machine, and then the oxygen-rich blood is returned to the patient's heart by the jugular vein. ECMO replaces the cardiopulmonary function of the COVID-19 patients with respiratory failure and heart failure to perform blood purification and oxygenation, so that the patient's heart and lungs can rest and gain time for self-healing. Long-term use of ECMO can cause serious side effects in patients, such as abnormal coagulation function, impaired renal function, impaired circulatory function (decreased tissue perfusion), and impaired respiratory function (perfused lung). The user fee of ECMO is also very high. Since ECMO must be used in the ICU, and the daily ICU user fee is also very considerable (about 100,000 yuan). Small town hospitals are hardly equipped with the expensive equipment.

In clinical practice, bronchoscopic balloon dilatation is the most common interventional method of the respiratory tract to treat bronchial stenosis. In this method, a special three-stage dilatation balloon catheter is inserted into the narrowed airway through a bronchoscope for step-by-step dilatation. It is mainly used to treat surgical scars, anastomotic stoma after lung transplantation, congenital dysplasia, granulomatous disease, and bronchial stenosis due to endotracheal intubation and other reasons. A number of clinical data have proved that the postoperative dilatation rate of balloon dilatation for benign bronchial stenosis is 90%-100%, and the restenosis rate after 6 months is less than 20%.

Bronchoalveolar lavage is a new technique developed on the basis of fiberoptic bronchoscopy by injecting normal saline into the lungs through a bronchoscope and aspirating the lavage fluid with negative pressure, and collecting alveolar surface fluid for identification and diagnosis of various pulmonary diseases, such as alveolitis, pulmonary fibrosis, asbestosis, lung cancer, pneumocystis, and pulmonary alveolar proteinosis. In recent years, it has also been gradually used in the treatment of patients with severe respiratory failure. The clinical manifestations of patients after surgery have improved with significantly increased oxygen saturation and oxygen partial pressure, and significantly decreased carbon dioxide partial pressure.

Interventional procedure has not been reported in the current treatment of COVID-19 patients. This is because any single device used for pulmonary interventional procedure in the world cannot meet the needs of the complex and rapidly evolving course of patients with severe COVID-19 due to its limited functions. The latest autopsy results show that the main reason for the poor treatment effect is that a large amount of viscous inflammatory secretions in the lung tissue accumulate in the bronchi and on the surface of the membranes at the bottom of the lungs, which obstructs the normal membrane oxygenation. Therefore, quickly and effectively clearing the "sludge" on the bronchi and lung membranes is the key to the emergency treatment of COVID-19. Although bronchoscopy and conventional sputum suction techniques can be used to clear and remove sputum, they are limited to trachea and have a single function. They are basically ineffective for the cytokine storm in the lungs of COVID-19 patients. Therefore, there is an urgent clinical need for a multifunctional device integrating tracheal dilatation, sputum excretion, alveolar lavage, continuous oxygen supply, nebulization and drug administration for the emergency treatment of COVID-19 patients.

### SUMMARY OF THE INVENTION

In view of the deficiencies of the prior art, an object of the present invention is to provide a multifunctional balloon dilatation catheter for intracorporeal membrane oxygenation and usage method therefor.

To achieve the above object, the present invention adopts the following technical scheme: The multifunctional balloon dilatation catheter for intracorporeal membrane oxygenation comprises a catheter hub located at the proximal end of the catheter, a dilatation balloon located at the distal end of the catheter and M channels located in the catheter. M channels comprise at least a balloon dilatation channel, a positioning & detection channel, a supply channel and a secretion removal channel. M is an integer greater than or equal to four. The catheter hub is a multi-channel connection device, through which M channels are respectively connected to the external therapeutic devices of the catheter;

The catheter is inserted into a bronchiole of the lungs. The balloon dilatation channel is used for dilating the narrowed part of the airway. The positioning & detection channel is used for positioning or detecting while inserting. The supply channel is used for continuous oxygen supply or drug administration to the patient. The secretion removal channel is used for removing secretions from the airway.
Further, the balloon dilatation channel is connected to the balloon inflation device through the catheter hub, the dilatation balloon has a dilatation port;
the secretion removal channel is connected to a sputum aspirator or a pressure syringe through the catheter hub, and has a lavage port;
the supply channel is connected to the supply device through the catheter hub, and has a supply port;
and the positioning & detection channel runs through both ends of the catheter, and can be used for guiding wire, biopsy forceps or fiber optic endoscope to pass through.

Further, the cross-sectional area of the secretion removal channel is larger than that of the balloon dilatation channel and the supply channel. Further, the positioning & detection channel is a cylindrical channel with an inner diameter greater than 0.1 mm and less than or equal to 5.0 mm.

Further, the dilatation balloon is a 3-stage dilatation balloon with the dilatation pressure within the range of 3-18 atm, and the outer diameter of 3-24 mm under inflation.

Further, the dilatation balloon is a single-stage dilatation balloon with the dilatation pressure within the range of 3-30 atm, and the outer diameter of 3-30 mm under inflation.

Further, the catheter is a catheter made from a polymer material, which is the same as the isolation material of M channels inside the catheter.
Further, a Luer connector is connected to the catheter hub, and the Luer connector and the catheter hub are used for connecting M channels and corresponding external therapeutic devices.

A method for intracorporeal membrane oxygenation using the catheter includes the following steps:
S01, inserting the catheter into a bronchus of the lungs through endotracheal intubation or through an airway endoscopy, the positioning & detection channel is used for guiding the catheter to advance during inserting, and the balloon dilatation channel controls the balloon to dilate the narrowed part;
S02, after the catheter reaches the designated position, using the supply channel to supply oxygen or medicine to the designated position; and
S03, after the catheter reaches the designated position, using the secretion removal channel to perform lavage and sputum suction in the designated position.

The beneficial effects of the present invention are as follows: the present invention integrates the dilatation balloon and the multi-lumen catheter, and presents a small outer diameter, so that the catheter can be directly inserted into a bronchus under the guidance of a bronchoscope or a fiber optic endoscope. During emergency treatment, on the one hand, the blocked airway is opened up by the dilatation of the dilatation balloon; on the other hand, the inflammatory excretions on the surface of the membranes can be aspirated and washed through the secretion removal channel to restore the oxygenation function of the membranes. Continuous oxygen supply using the supply channel, nebulization therapy or artificial ventilator are used to ensure maximum oxygen supply for patients, so as to achieve the emergency treatment of all sites (bronchi, bronchioles, and alveoli) and all directions (tracheal dilatation, sputum excretion, oxygen supply and drug administration) using a single catheter. This product is also suitable for the treatment of pneumonia caused by various reasons (such as mycoplasma pneumonia, airway stenosis and pneumonia caused by tuberculosis), silicosis, etc.

### BRIEF DESCRIPTION OF THE DRAWINGS

Fig. 1 is the structural schematic diagram of the catheter of the present invention;
Fig. 2 is the schematic diagram of each channel in the catheter of the present invention;
Fig. 3 is the schematic diagram of the balloon part in the catheter of the present invention;
Fig. 4 is the schematic diagram of the position of the balloon when the catheter in the present invention is inserted into the lower respiratory tract;
Fig. 5 is the physical diagram of the catheter hub in the present invention;
Fig. 6 is the physical diagram of the catheter in the present invention.

Reference numerals: 1. Secretion removal channel; 2. Supply channel; 3. Balloon dilatation channel; 4. Positioning & detection channel; 5. Catheter hub; 6. Dilatation balloon; 7. Catheter end; 8. Supply port; 9. Dilatation port; 10. Lavage port; 11. Trachea; 12. Main bronchus; 13. Bronchiole.

### DETAILED DESCRIPTION OF THE INVENTION

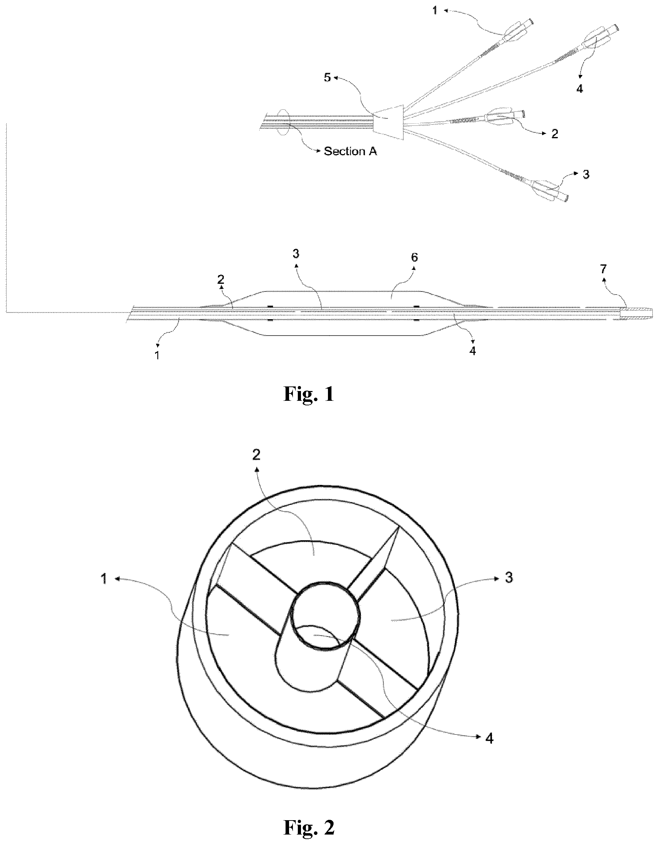

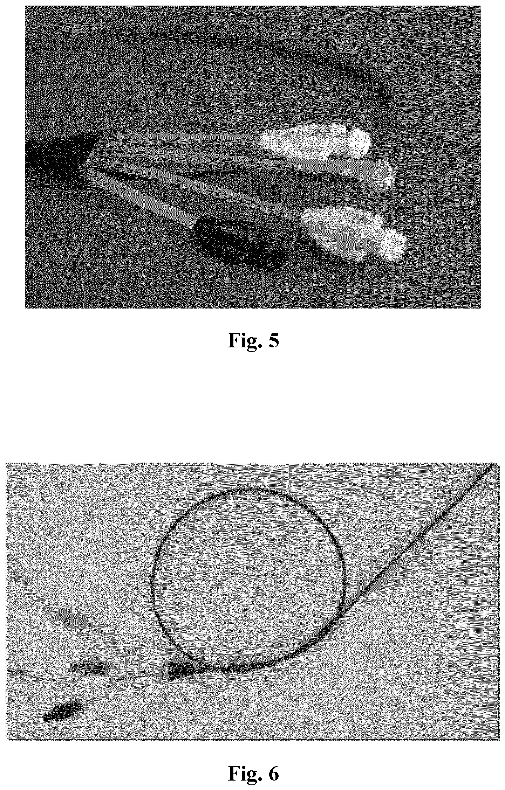

The invention will be further described below with reference to the drawings and specific embodiments:
Referring to Figs. 1-5, a multifunctional balloon dilatation catheter for intracorporeal membrane oxygenation comprises a catheter hub 5 located at the proximal end of the catheter, a dilatation balloon 6 located at the distal end of the catheter and M channels located in the catheter. The catheter hub is a multi-channel connection device, through which M channels are respectively connected to the external therapeutic devices of the catheter. As shown in Fig. 5 , a Luer connector can also be connected to the catheter hub. The Luer connector and the catheter hub are used for connecting M channels and corresponding external therapeutic devices, and enable switching between the external corresponding therapeutic devices. In the present invention, M channels comprise at least a balloon dilatation channel 3, a positioning & detection channel 4, a supply channel 2 and a secretion removal channel. M is an integer greater than or equal to four. Fig. 6 is the physical diagram of the catheter in the present invention comprising four channels. In the specific embodiments, the number of channels and their functions can be increased or replaced according to the function of the catheter and the purpose of treatment, which can cover channels with any function during the treatment of bronchioles.

The balloon dilatation channel is connected to the balloon inflation device through the catheter hub. The balloon dilatation channel 3 is communicated with the dilatation balloon 6, which has a dilatation port. The balloon inflation device inflates the gas to the dilatation balloon via the balloon dilatation channel and the dilatation port, so that the dilatation balloon 6 is dilated under certain conditions to open the blocked or narrowed part in the human body, so as to advance the catheter smoothly.

The positioning & detection channel in the present invention is used for guiding during insertion, and it can also be used for biopsy or fiberoptic endoscope insertion.

Specifically, the positioning & detection channel 4 can be used as a guide wire channel, a biopsy channel, or an endoscopic channel. A guide wire can be inserted into the positioning & detection channel 4 to guide the insertion of the catheter. A visualization system can also be connected to the positioning & detection channel 4 through the catheter hub to insert the catheter under the instruction. Biopsy forceps can also be inserted into the positioning & detection channel 4, which is connected to the detection system through the catheter hub, and the lung tissue sample is obtained after the catheter is inserted into the lung. The positioning & detection channel is a cylindrical channel with an inner diameter greater than 0.1 mm and less than or equal to 5.0 mm. When the positioning & detection channel 4 is connected to the visualization system, it is used for navigating the advancing of the catheter (direction and position). When the positioning & detection channel 4 is connected to the detection system, it is used for removing a piece of tissue at the distal end of the catheter, so as to know the condition of the patient at any time.

The supply channel is connected to the supply device through the catheter hub. The supply device can be an oxygen source or a drug source. The supply channel has a supply port, which is generally provided at the proximal end of the catheter. The supply channel and the supply port are used for oxygen supply and/or drug administration. Specifically, the supply channel can nebulize oxygen and then spray it into the bronchi accurately. For patients who require drug therapy, the drug can be nebulized together with oxygen for topical administration to minimize the damage caused by the drug to the system.

The secretion removal channel 1 is connected to a sputum aspirator or a pressure syringe through the catheter hub. It is used for bronchial or alveolar lavage and sputum aspiration.

The catheter of the present invention and the channels inside the catheter can be of any shape, as long as the purpose of treating the bronchioles in the present invention can be achieved. As a preferred embodiment, the example in Fig. 2 is a schematic diagram of the internal section corresponding to Section A in Fig. 1: The catheter can be provided as a cylindrical catheter, the positioning & detection channel 4 is a cylindrical channel, and the secretion removal channel 1, the supply channel 2 and the balloon dilatation channel 3 are distributed between the positioning & detection channel 4 and the outer surface of the catheter, similar to a fan-shaped distribution. The cross-sectional area of the secretion removal channel 1 is generally larger than that of the balloon dilatation channel 3 and the supply channel 2. The lavage port 10, the dilatation port 9 and the supply port 8 are all located on the outer surface of the catheter.

In the present invention, the inner diameter of the positioning & detection channel 4 is greater than 0.1 mm and less than or equal to 5.0 mm, so that the guide wire with a diameter of more than 0.05 mm and less than 5.0 mm can pass through the positioning & detection channel. A visualization system is connected at the starting end of the positioning & detection channel to observe the position of the catheter in the human body at any time. The visualization system and the positioning & detection channel are used for navigating the insertion and positioning of the catheter, or a fiber optic endoscope is embedded in the positioning & detection channel to guide the insertion of the catheter. The positioning & detection channel 4 can also be connected to a detection device through the catheter hub to remove a piece of tissue at the distal end of the catheter, so as to know the condition of the patient at any time.

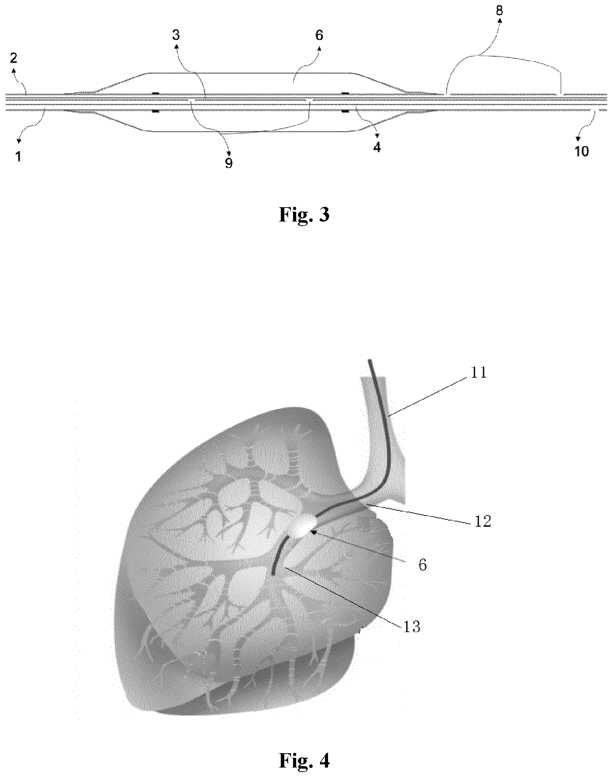

Referring to Fig. 3, the balloon dilatation channel 3 also has a dilatation port 9, which is located inside the balloon 6, and an air pump is connected at the front end of the balloon dilatation channel 3. The balloon 6 is located in the middle of the catheter near the end. The outer diameter of the balloon is larger than that of other parts of the catheter under inflation. The balloon can be dilated to different sizes under different inflation pressures for the purpose of dilating a narrowed or blocked part of the body. In practice, the dilatation balloon may be a 3-stage dilatation balloon with the dilatation pressure within the range of 3-18 atm, and the outer diameter of 3-24 mm under inflation. The dilatation balloon may also be a single-stage dilatation balloon with the dilatation pressure within the range of 3-30 atm, and the outer diameter of 3-30 mm under inflation. The position and number of the dilatation ports in the balloon can be designed according to specific requirements.

The catheter in the present invention can be, but is not limited to, a polymer catheter, and the isolation material of M channels inside the catheter is the same as the catheter material. The hardness of the polymer catheter is higher than that of the balloon, so that the guide wire leads the catheter, and the catheter leads the ball to a bronchus of the lungs. Preferably, the balloon in the present invention comprises two bridging portions, a gradual portion and a cylindrical portion. The bridging portions are located at both ends of the balloon and fixedly connected to the outer surface of the catheter. The gradual portion connects the bridging portion and the cylindrical portion, the outer diameter of the gradual portion increases successively from the bridging portion to the cylindrical portion, and the outer diameter of the cylindrical portion is larger than that of the bridging portion. The outer diameter of the bridging portion is the same as that of the catheter, and the outer diameter of the cylindrical portion needs to be larger than that of the catheter. The specific size can be determined according to the inflation state of the balloon. When the balloon is not inflated, the outer diameter of the balloon only needs to be slightly larger than that of the catheter. In order to ensure a better opening effect of the balloon during the insertion of the catheter into the bronchiole, the length of the bridging portion: the length of the gradual portion: the length of the cylindrical portion should be 1:2:7 in the advancing direction of the catheter. This length ratio can ensure that the balloon is firmly fixed to the outer surface of the catheter. When the narrowed part is opened, the gradual portion can play a buffer role to the narrowed part to avoid human tissue damage due to the dash of the cylindrical portion on the narrowed part.

The function of the balloon is to open the narrowed or blocked part of the human body. During the normal advancement of the catheter, the balloon does not need to be inflated. Only when an obstacle or a narrowed part is encountered during the advancement of the catheter, it is necessary to inflate the balloon using the air pump. Since the dilatation port is located inside the balloon, the balloon can be dilated by inflation to open the narrowed or blocked part, so that the catheter can continue to advance. The balloon may be made from materials including but not limited to nylon and its copolymers, polyethylene terephthalate (PET), polyether block polyamide (PEBAX) and one of other polymers or copolymers.

Referring now to Fig. 3, the supply channel 2 also has a supply port 8, which is located at the front and/or rear end of the balloon in the catheter. The oxygen source is connected at the front end of the supply channel 2. A mechanical ventilator may be connected at the front end of the supply channel to deliver nebulized oxygen to specific parts of the human body. A drug source may also be connected at the front end of the supply channel to supply the drug to the lungs. For patients who require drug therapy, the drug can be nebulized together with oxygen for topical administration to minimize the damage caused by the drug to the system. The position and number of the supply ports can be designed according to specific requirements. Usually, supply ports must be provided at the lower end of the balloon, and supply ports can be selectively provided at the upper end of the balloon.

Referring now to Fig. 3, the secretion removal channel 1 also has a lavage port 10, which is located at the distal end of the balloon in the catheter. A sputum aspirator or a pressure syringe is connected at the front end of the secretion removal channel for the purpose of lavage and sputum aspiration. When there is inflammation at the site where the catheter reaches, there may be inflammatory secretions, etc. The secretions can be aspirated from the body by the sputum aspirator and through the secretion removal channel, and the cleaning solution such as normal saline can be pumped into the designated part through the secretion removal channel for lavage. Timely lavage of the inflammatory sites of patients with COVID-19, and removal of fluid in the bronchi and alveoli can effectively alleviate the respiratory distress due to the cytokine storm.
In order to keep the distal end of the catheter better inserted into the human body, the distal end 7 of the catheter can be designed with a suitable hardness, which will not damage the human tissue, and can be smoothly inserted into the bronchioles under the guidance of a guide wire or a fiberoptic endoscope. The perimeter of the outer diameter of the catheter is about 2-10 mm. As shown in Fig. 4, the catheter can be easily passed through the trachea 11 and the main bronchus 12 and enter any bronchiole 13 of the lungs or alveolus through endotracheal intubation or through an airway endoscopy at the bedside under the guidance of the guide wire or fiber optic endoscope at the bedside due to its small outer diameter. The insertion is less invasive and easy to operate, which not only shortens the exposure time of medical staff to the viruses, but also reduces the pain of patients caused by intubation. The catheter is especially suitable for bronchiolar dilatation.

A method for intracorporeal membrane oxygenation using the catheter includes the following steps:
S01, inserting the catheter into a bronchus of the lungs through endotracheal intubation or through an airway endoscopy, the guide wire in the positioning & detection channel is used for guiding the catheter to advance and the balloon in the balloon dilatation channel is used for dilating the narrowed part;
S02, after the catheter reaches the designated position, using the oxygen source to nebulize and supply oxygen to the designated position through the supply channel, so as to spray the nebulized oxygen into the bronchi accurately, and the drug can be nebulized together with oxygen for topical administration to minimize the damage caused by the drug to the system; and
S03, after the catheter reaches the designated position, using a sputum aspirator or a pressure syringe to connect to the secretion removal channel for lavage and removal of inflammatory secretions, and timely lavage of the inflammatory sites of patients with COVID-19 and removal of fluid in the bronchi and alveoli can effectively alleviate the respiratory distress due to the cytokine storm.

The present invention integrates the dilatation balloon and the multi-lumen catheter, and presents a small outer diameter, so that the catheter can be directly inserted into a bronchiole under the guidance of a bronchoscope or a fiber optic endoscope. During emergency treatment, on the one hand, the blocked airway is opened up by the dilatation and contraction of the dilatation balloon; on the other hand, the inflammatory excretions on the surface of the membranes can be aspirated and washed through the secretion removal channel to restore the oxygenation function of the membranes. Continuous oxygen supply using the supply channel, nebulization therapy or artificial ventilator are used to ensure maximum oxygen supply for patients, so as to achieve the emergency treatment of all sites (bronchi, bronchioles, and alveoli) and all directions (tracheal dilatation, sputum excretion, oxygen supply and drug administration) using a single catheter. This product is also suitable for the treatment of pneumonia caused by various reasons (such as mycoplasma pneumonia, airway stenosis and pneumonia caused by tuberculosis), silicosis, etc.

For those skilled in the art, various other corresponding changes and variants may be made according to the technical scheme and concepts described above, and all these changes and variants should fall within the protection scope of the claims of the present invention.

## Claims

1. A multifunctional balloon dilatation catheter for intracorporeal membrane oxygenation, comprising: a catheter hub located at the proximal end of the catheter, a dilatation balloon located at the distal end of the catheter, and M channels located in the catheter; M channels comprising at least a balloon dilatation channel, a positioning & detection channel, a supply channel and a secretion removal channel; M is an integer greater than or equal to four; the catheter hub is a multi-channel connection device, through which M channels are respectively connected to the external therapeutic devices of the catheter; and
the catheter is inserted into a bronchiole of the lungs, the balloon dilatation channel is used for dilating the narrowed part of the airway, the positioning & detection channel is used for positioning or detecting while inserting, the supply channel is used for continuous oxygen supply or drug administration to the patient, and the secretion removal channel is used for removing secretions from the airway.

2. The multifunctional balloon dilatation catheter for intracorporeal membrane oxygenation according to claim 1, wherein the balloon dilatation channel is connected to a balloon inflation device through the catheter hub, and the dilatation balloon has a dilatation port;
the secretion removal channel is connected to a sputum aspirator or a pressure syringe through the catheter hub, and has a lavage port;
the supply channel is connected to the supply device through the catheter hub, and has a supply port; and
the positioning & detection channel runs through both ends of the catheter, and can be used for guiding wire, biopsy forceps or fiber optic endoscope to pass through.

3. The multifunctional balloon dilatation catheter for intracorporeal membrane oxygenation according to claim 2, wherein the cross-sectional area of the secretion removal channel is larger than that of the balloon dilatation channel and the supply channel.

4. The multifunctional balloon dilatation catheter for intracorporeal membrane oxygenation according to claim 1, wherein the positioning & detection channel is a cylindrical channel with an inner diameter greater than 0.1 mm and less than or equal to 5.0 mm.

5. The multifunctional balloon dilatation catheter for intracorporeal membrane oxygenation according to claim 1, wherein the dilatation balloon is a 3-stage dilatation balloon with the dilatation pressure within the range of 3-18 atm, and the outer diameter of 3-24 mm under inflation.

6. The multifunctional balloon dilatation catheter for intracorporeal membrane oxygenation according to claim 1, wherein the dilatation balloon is a single-stage dilatation balloon with the dilatation pressure within the range of 3-30 atm, and the outer diameter of 3-30 mm under inflation.

7. The multifunctional balloon dilatation catheter for intracorporeal membrane oxygenation according to claim 1, wherein the catheter is a catheter made from a polymer material, which is the same as the isolation material of M channels inside the catheter.

8. The multifunctional balloon dilatation catheter for intracorporeal membrane oxygenation according to claim 1, wherein a Luer connector is connected to the catheter hub, and the Luer connector and the catheter hub are used for connecting M channels and corresponding external therapeutic devices.

9. A method for intracorporeal membrane oxygenation using the catheter according to claim 1, comprising the following steps:
S01, inserting the catheter into a bronchus of the lungs through endotracheal intubation or through an airway endoscopy. When inserting, the positioning & detection channel is used for guiding the catheter to advance, and the balloon dilatation channel controls the balloon to dilate the narrowed part;
S02, after the catheter reaches the designated position, using the supply channel to supply oxygen or medicine to the designated position; and S03, after the catheter reaches the designated position, using the secretion removal channel to perform lavage and sputum suction in the designated position.
